# EUROPÄISCHE PATENTSCHRIFT

(11) **EP 2 480 187 B1**
(45) Veröffentlichungstag und Bekanntmachung des Hinweises auf die Patenterteilung: **20.02.2019**
(21) Anmeldenummer: 10779205.3
(22) Anmeldetag: 09.09.2010
(51) Int. Cl.: A61F 9/013

(54) **SCHABLONE ZUM AUFLEGEN AUF EIN AUGE**
TEMPLATE FOR PLACING ON AN EYE
GABARIT À POSER SUR UN OEIL

(30) Priorität: 21.09.2009 DE 102009042009; 25.09.2009 US 277480 P; 30.11.2009 US 264978 P
(43) Veröffentlichungstag der Anmeldung: 01.08.2012
(73) Patentinhaber: Neuhann, Tobias, 80796 München (DE)
(72) Erfinder: Neuhann, Tobias, 80796 München (DE)
(74) Vertreter: Sattler de Sousa e Brito, Clara
(86) Internationale Anmeldenummer: PCT/DE2010/001058
(87) Internationale Veröffentlichungsnummer: WO 2011/032532

(56) Entgegenhaltungen:
- WO-A1-00/41660
- WO-A1-99/39238
- WO-A2-96/08212
- US-B1- 6 679 898

## Beschreibung

Die Erfindung betrifft eine Schablone zum Auflegen auf ein Auge.

Vorrichtungen zum Auflegen auf ein Auge in Form von Unterdrucksaugringen zur Straffung der Hornhaut sind aus dem Stand der Technik zahlreich bekannt. Diese sogenannten Mikrokeratome werden auf das Auge aufgesetzt und an den Saugring wird ein Unterdruck angelegt, wodurch die Hornhaut gestrafft wird. So kann diese dann weiter in dieser gerade gezogenen Form operativ und mittels einer Lasik Behandlung weiter behandelt werden.

Vorrichtungen zum Aufsetzen auf das Auge zur Verformung der Hornhaut sind auch aus der Orthokeratologie bekannt. Durch orthokeratologische Kontaktlinsen kann die Hornhaut über Nacht verformt werden, was zu einer Brechkraftkorrektur und somit zu einer korrigierten Sehkraft unter Tag führt. Die dazu verwendeten harten Kontaktlinsen sind auf der Außenseite glatt und auf der Innenseite entsprechend der gewünschten Brechkraftkorrektur geformt. Dadurch wird die Hornhaut in die gewünschte Form geschoben.

Aufgabe der vorliegenden Erfindung ist es, eine verbesserte Schablone zum Auflegen auf ein Auge zur Verfügung zu stellen.

Nach einem ersten Aspekt der Erfindung löst diese Aufgabe eine Schablone zum Auflegen auf ein Auge, wobei sie an einer Seite eine kreisförmige Einbuchtung aufweist. Diese Einbuchtung entspricht der gewünschten berechneten korrigierten Hornhautform, die zu einer Korrektur einer Fehlsichtigkeit notwendig ist. Die Schablone wird an die zu behandelnde Fehlsichtigkeit angepasst. Eine Behandlung von Kurzsichtigkeit, Weitsichtigkeit, Presbyopie und Astigmatismus ist möglich. Eine Anpassung an individuelle Abberationen des zu behandelnden Auges, die beispielsweise im Rahmen einer Wellenfrontanalyse bestimmt wurden, kann vorgenommen werden.

Eine solche Schablone kann aufs Auge aufgesetzt werden. Dann werden die Druckverhältnisse zwischen dem Auge und dem zwischen der Hornhaut und der Einbuchtung gebildeten Hohlraum so verändert, dass die Hornhaut in die Einbuchtung gedrückt wird. Dies kann entweder dadurch erzielt werden, dass an die Einbuchtung ein Unterdruck angelegt wird, oder dadurch erzielt werden, dass ein Viskoelastikum in das Augeninnere gespritzt wird, wodurch sich der Augeninnendruck erhöht.

Vorteilhaft ist, wenn die Schablone um die erste Einbuchtung eine zweite Einbuchtung in Form eines ringförmig gewölbten Bereichs aufweist. Dieser kann entweder so geformt sein, dass er an die präoperative Form der Hornhaut in diesem Bereich spiegelbildlich angepasst ist. So dient er beim Auflegen der Schablone für Abdichtung. Eine zweite Möglichkeit besteht darin, diese zweite Einbuchtung so zu formen, dass sie ebenfalls einer Sehkorrektur dient. In diesem Fall ist auch diese Einbuchtung so geformt, dass sie der postoperativ gewünschten Hornhautkrümmung entspricht.

Von Vorteil ist, wenn die Schablone einen Kanal aufweist, der mit der Einbuchtung verbunden ist. Durch diesen Kanal kann die Luft aus dem durch Einbuchtung und Hornhaut gebildeten Hohlraum entfernt werden.

Dies kann erfolgen, indem an dem Kanal eine Vorrichtung zum Erzeugen von Unterdruck angeordnet ist. Eine solche Vorrichtung kann beispielsweise eine Unterdruckpumpe oder eine von Hand zu bedienende Saugglocke oder ein Ballon sein. Diese erzeugen den Unterdruck in dem von Hornhaut und Einbuchtung gebildeten Hohlraum. Dieser kann variiert und für eine bestimmte Zeit gehalten werden.

Um einen konstanten Unterdruck erzeugen zu können, kann die Seite der Schablone, an der sich die Einbuchtung befindet, am Rand eine Dichtung aufweisen. Diese schließt mit der Hornhaut ab und verhindert so eine Verringerung des Unterdrucks. Diese kann beispielsweise in Form eines Silikonrings ausgestaltet sein.

Die Schablone kann zylinderförmig sein. Diese Form bietet sich auf Grund der kugelförmigen Augengeometrie an.

Sie kann einen Durchmesser von 2 bis 20 mm aufweisen. Während für die Behandlung der Altersweitsichtigkeit (Presbyopie) kleine Durchmesser von 2 bis 4 mm möglich sind, sind für die Behandlung der Weitsichtigkeit oder Kurzsichtigkeit größere Durchmesser von bis zu 20 mm abhängig vom zu behandelnden Hornhautbereich notwendig. Zusätzlich zu dem zu behandelnden Hornhautbereich kann noch ein Randbereich notwendig sein, der auf einem nicht zu behandelnden Teil der Hornhaut zur Abdichtung oder zur besseren Auflage aufliegt.

Die Einbuchtung selbst kann einen Durchmesser von 2 bis 15 mm aufweisen. Dieser entspricht dem zu behandelnden Hornhautbereich.

Zur Behandlung der Altersweitsichtigkeit ist es hilfreich, wenn die erste Einbuchtung eine Tiefe von 2 bis 8 µm aufweist. Dies entspricht der in der Regel notwendigen Korrektur der Hornhautkrümmung.

Die Schablone kann aus unterschiedlichen Materialien gefertigt sein. So sind beispielsweise verschiedene Kunststoffe, wie PMMA oder Silikon denkbar. Diese Materialien haben den Vorteil, dass die Schablonen in Form von Disposibles hergestellt werden können, d.h. sie werden je Patient nur einmal zur Behandlung verwendet und danach weggeworfen. Die Schablonen können in ihrer Form an den jeweiligen Patienten angepasst sein. Die Herstellung aus Kunststoff hat hierbei den Vorteil, dass sie besonders leicht und kostengünstig möglich ist.

Ein weiteres denkbares Material wäre Titan. Eine Herstellung aus Titan hätte den Vorteil, dass die Schablone ein geringes Gewicht hat und wiederverwendbar wäre. Insbesondere ist eine Sterilisierung möglich.

Auch andere Materialien, wie beispielsweise Stahl, sind denkbar.

Ein weiterer Aspekt der Erfindung, der auch alleine erfindungswesentlich ist, betrifft eine Schablone zum Auflegen auf ein Auge, wobei die Schablone eine Massageeinrichtung aufweist. Mit dieser kann die Hornhaut des Auges in bestimmten Bereichen massiert werden.

Vorteilhafterweise ist die Massageeinrichtung kreisförmig und damit an die Augengeometrie angepasst. Sie kann beispielsweise stempelförmig sein und so angeordnet sein, dass sie mittig auf den Augapfel über der Pupille drückt. Sie kann einen Durchmesser von 2 mm bis 20 mm aufweisen.

Die Massageeinrichtung kann mindestens ein Ringelement aufweisen. Insbesondere kann sie auch vollständig ringförmig ausgestaltet sein. Dies hat den Vorteil, dass die Massageeinrichtung um eine Schablone mit einer kreisförmigen Einbuchtung aufgesteckt werden kann. Auch eine alleinige Verwendung der Massageeinrichtung ist jedoch denkbar.

Bei einer kombinierten Verwendung von Massageeinrichtung und Schablonenteil mit Einbuchtung ist es möglich den Teil mit der Einbuchtung als kostengünstigeres Verschleißteil auszubilden und die Massageeinrichtung als dauerhafte, wiederverwendbare Vorrichtung auszugestalten. Dies ist insbesondere deshalb sinnvoll, da zu erwarten ist, dass die Massageeinrichtung technisch deutlich komplexer sein wird und damit das deutlich teurere Bauteil darstellt.

Ist an dem Auge in einem vorgelagerten Schritt eine Behandlung mit einem Laser erfolgt insbesondere mit einem Femtosekunden-Laser, können die Ringelemente an die Größe und Form des Lasermusters angepasst sein. Bei einem ringförmigen Lasermuster ist es sinnvoll die Ringelemente so anzuordnen, dass sie zwischen den Laserschnitten zu liegen kommen. Diese können dann beim inneren Ringelement einen Innendurchmesser des Rings von ungefähr 1,3 mm bis 2 mm haben. Der Abstand der einzelnen Ringelemente kann in der Größenordnung von 0,1 mm bis 0,4 mm liegen. Auch die Dicke der Ringelemente kann in diesem Bereich angeordnet sein. Da das verwendete Muster des Lasers unabhängig vom Patienten immer identisch sein wird, ist die Verwendung einer einzelnen Massageeinrichtung für verschiedene Patienten möglich.

Durch die Massageeinrichtung können Drücke in der Größenordnung von 10 mm Hg bis 100 mm Hg auf die Hornhaut angelegt werden. Eine Auslenkbarkeit von 10 µm bis 1 mm der Ringelemente ist vorteilhaft.

Die einzelnen Ringelemente können mit Federn versehen werden. Wird auf ein Ringelement gedrückt, dehnt sich die Feder. Wird der Druck wieder entfernt, bewegt sich die Feder in einen Ruhezustand zurück.

Die Massageeinrichtung kann auch mehrere Ringsegmente aufweisen. Dies ist insbesondere dann sinnvoll, wenn bei einer vorgelagerten Laserbehandlung ein radiäres Schnittmuster zum Einsatz gekommen ist. Die Ringsegmente können in einem Ringelement insbesondere in der Größenordnung von 10 mm bis 15 mm Innendurchmesser angeordnet sein. Dieses kann insbesondere eine Breite von 2 mm bis 5 mm aufweisen. Die Breite eines Ringsegments kann ebenfalls 2 mm bis 5 mm betragen.

Sowohl die Ringelemente als auch die Ringsegmente können gegeneinander bewegt werden. Insbesondere können dabei benachbarte Elemente gegensätzlich bewegt werden. Auch eine wellenförmige Bewegung der verschiedenen Elemente ist jedoch denkbar.

Die Massageeinrichtung kann automatisiert sein. So können komplexe Bewegungsmuster der Elemente zueinander in kurzer Zeit ausgeführt werden. Außerdem ist eine Standardisierung der Verwendung der Massageeinrichtung möglich. Auch eine manuelle Bedienung der Massageeinrichtung ist jedoch denkbar.

Ein weiterer Aspekt der nicht Teil der Erfindung ist, betrifft eine Folie zum Auflegen auf das Auge, insbesondere zwischen das Auge und einer erfindungsgemäßen Schablone, wobei die Schablone aus Acryl, insbesondere hydrophilem Acryl oder Silikon sein kann. Eine solche Folie hat den Vorteil, dass die bei der Massage auftretenden Druck- und Scherkräfte nicht direkt auf die Hornhaut wirken. Dadurch kann eine Verletzung der Hornhaut, insbesondere der oberen Zellschichten vermieden werden. Gleichzeitig muss die Folie dünn und flexibel genug sein um eine ausreichende Weitergabe von Druckänderungen entweder durch Massage oder die Erzeugung eines Unterdrucks an die Hornhaut zu ermöglichen. Die Folie kann ebenfalls kreisförmig sein und so an die Geometrie der Schablone angepasst werden. Es ist auch denkbar, dass die Schablone bereits eine Vorrichtung aufweist, in die die Folie eingespannt werden kann.

Ein weiterer Aspekt der nicht Teil der Erfindung ist, betrifft ein Verfahren zur Herstellung einer derartigen Schablone, wobei eine erste Einbuchtung entsprechend einer gewünschten Sehkorrektur geformt ist. Dabei wird die gewünschte Hornhautkrümmung berechnet. Die Einbuchtung wird entsprechend spiegelbildlich der gewünschten und berechneten Hornhautkrümmung geformt.

Dies kann sowohl für die erste Einbuchtung alleine als auch für eine Kombination aus der ersten Einbuchtung und der zweiten Einbuchtung geschehen.

Schließlich kann die Herstellung der zweiten Einbuchtung anhand von Daten einer 3D-Darstellung einer Augenoberfläche eines Patienten geformt werden. Dies hat den Vorteil, dass die Schablone passgenau auf der Hornhaut des Patienten aufliegt.

Ein letzter Aspekt der nicht Teil der Erfindung ist, betrifft schließlich die Verwendung einer derartigen Schablone, wobei in einem ersten Schritt die Schablone aufs Auge aufgesetzt wird und in einem zweiten Schritt das Druckverhältnis zwischen dem Auge und dem durch die Einbuchtung gebildeten Hohlraum so angepasst wird, dass in der Einbuchtung ein Unterdruck entsteht.

So wird die Hornhaut in die Einbuchtung gedrückt. Dies kann entweder dadurch geschehen, dass der Druck im Auge erhöht wird oder der Druck im Holraum verringert wird.

Das Verfahren kann einen zwischengelagerten Schritt aufweisen, in dem das Auge massiert wird. Auch dadurch kann ein Druck bzw. Zug auf die Hornhaut ausgeübt werden. Dieser kann daher auch den zweiten Schritt vollständig ersetzen.

In einem vorgelagerten Schritt kann eine Behandlung der Fehlsichtigkeit mit einem Laser erfolgen. Insbesondere kann in einem vorgelagerten Schritt das Stoma der Hornhaut mittels eines Femtosekunden-Lasers eingeschnitten werden, ohne dass die darüber und darunter liegenden Membranen beschädigt werden. So kann die Hornhaut anschließend leicht und dauerhaft in die durch die Schablone vorgesehene gewünschte Form gezogen werden.

In einem weiteren noch vorgelagerten Schritt kann eine erfindungsgemäße Folie auf das Auge aufgelegt werden. Diese schützt die Hornhaut vor Verletzungen.

Die Erfindung wird nachfolgend anhand dreier Ausführungsbeispiele unter Bezugnahme auf die Zeichnung näher erläutert.

Hierin zeigen
- Fig. 1: eine schematische dreidimensionale Darstellung einer Schablone,
- Fig. 2: einen möglichen Querschnitt durch eine Schablone und
- Fig. 3: einen Querschnitt durch eine Schablone zur Presbyopiekorrektur bei gleichzeitiger Korrektur der Weit-bzw. Kurzsichtigkeit und

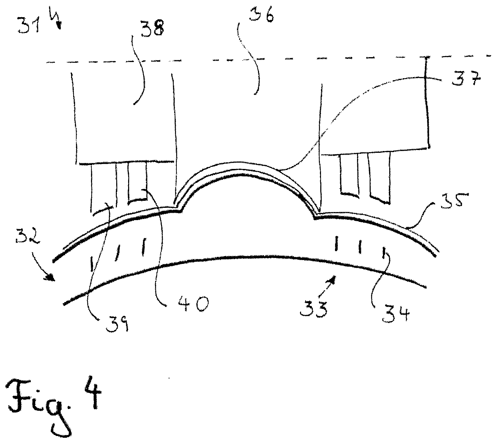

- Fig. 4: einen schematischen Querschnitt durch einen Ausschnitt einer Schablone mit einer Massageeinrichtung auf einer mit Femtosekunden-Laser behandelten Hornhaut.

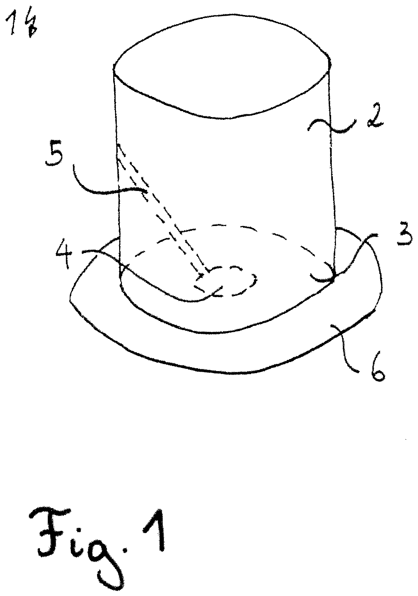

Die Schablone 1 in Figur 1 besteht im Wesentlichen aus einem Schablonengrundkörper 2, an dessen Unterseite 3 sich eine Einbuchtung 4 befindet. Zu dieser Einbuchtung 4 läuft ein Kanal 5, mittels dem ein Unterdruck an die Einbuchtung angelegt werden kann. Eine solche Schablone wird mittels der unteren Seite 3 auf ein Auge aufgesetzt. Wird dann mittels einer geeigneten Vorrichtung (nicht abgebildet) wie einer Unterdruckpumpe oder einem Ballon ein Unterdruck an die Einbuchtung 4 angelegt, so wird die Hornhaut in die Einbuchtung 4 gezogen.

Zur besseren Abdichtung kann die Unterseite 3 in dem Bereich um die Einbuchtung so ausgestaltet sein, dass sie an die Augenoberfläche angepasst ist und möglichst passgenau mit dieser abschließt. Bei einer zweiteiligen Behandlung im Bereich der Einbuchtung 4 und im Bereich des ringförmigen Teils der Unterseite 3 um die Einbuchtung 4 ist es möglich, dass auch dieser ringförmige Teil der Unterseite 3 eine entsprechende Wölbung aufweist. Sowohl die Wölbung dieses Bereichs als auch die der Einbuchtung 4 verhalten sich spiegelbildlich zu der durch die Behandlung zu erzielenden erwünschten Hornhautkrümmung.

Um eine bessere Abdichtung beim Anlegen des Unterdrucks zu erzielen, ist es weiter möglich, dass die Schablone 1 als Dichtung einen Dichtring 6 aufweist. Dieser kann beispielsweise aus Silikon sein.

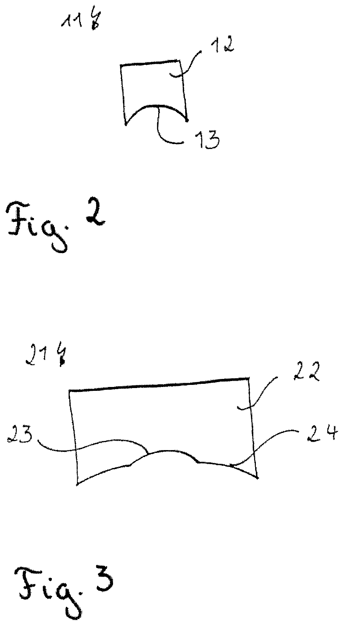

Der in Figur 2 dargestellte schematische Querschnitt durch eine Schablone 11 zeigt eine mögliche Ausgestaltung des Schablonengrundkörpers 12, dessen untere Seite fast vollständig aus einer Einbuchtung 13 besteht.

Bei dem in Figur 3 dargestellten schematischen Querschnitt durch eine Schablone 21 ist der Schablonengrundkörper 22 so ausgestaltet, dass neben einer ersten Einbuchtung 23 ein zweiter ringförmiger gewölbter Bereich 24 vorgesehen ist. Dabei entspricht der erste Bereich 23 in der Form spiegelbildlich der gewünschten Hornhautkrümmung in diesem Bereich. Der zweite Bereich 24 kann entweder an die präoperative Form der Hornhaut des Patienten vor der Behandlung angepasst werden und dient dann nur der Abdichtung. Es ist jedoch auch möglich auch den zweiten Bereich spiegelbildlich in Form einer nach der Operation zu erzielenden Hornhautkrümmung zu formen. Dann wird die Hornhaut auch in diesem Bereich durch Unterdruckerzeugung in die gewünschte Form gezogen.

Die Schablone 31 ist auf eine laserbehandelte Hornhaut 32 mit einem ringförmigen Laserschnittmuster 33 mit mehreren ringförmigen Schnitten wie beispielsweise dem Schnitt 34 aufgesetzt. Zwischen Schablone 31 und Hornhaut 32 befindet sich die Folie 35. Die Schablone 31 besteht aus einem inneren Teil 36 mit einer Einbuchtung 37. Um diese herum befindet sich ein ringförmiger zweiter Teil, die Massageeinrichtung 38. Diese weist mehrere Ringsegmente 39 und 40 auf. Diese sind in ihrer Größe auf das Schnittmuster abgestimmt, sodass sie zwischen den Schnitten zu liegen kommen. Der innere Teil der Schablone 36 weist eine Breite von ca. 1,6 mm auf. Der Abstand von der Mitte eines der Ringelemente 39 zum nächsten Ringelement 40 entspricht etwa 2 mm. Werden die Ringelemente 39, 40 gegeneinander bewegt, erfolgt eine Massage der Hornhaut 32. Wird anschließend ein Unterdruck an die Einbuchtung 37 angelegt, kann durch die Lockerung der Schnitte wie beispielsweise 34 die Hornhaut 32 leichter in die Einbuchtung 37 gezogen werden.

## Patentansprüche

1. Schablone zum Auflegen auf ein Auge, die an einer Seite eine kreisförmige Einbuchtung (4) aufweist, die entsprechend einer gewünschten Sehkorrektur geformt ist, **dadurch gekennzeichnet, dass** die Schablone (1) einen Kanal (5) aufweist, der mit der Einbuchtung verbunden ist und an dem Kanal eine Vorrichtung angeordnet ist, welche einen Unterdruck erzeugt, der die Druckverhältnisse zwischen dem Auge und der Schablone in einem zwischen der Hornhaut und der Einbuchtung gebildeten Hohlraum, wenn die Schablone auf dem Auge aufliegt, so verändert, dass die Hornhaut in die Einbuchtung gedrückt bzw. gezogen wird.

2. Schablone nach Anspruch 1, **dadurch gekennzeichnet, dass** sie um die erste Einbuchtung eine zweite Einbuchtung in Form eines ringförmig gewölbten Bereichs aufweist.

3. Schablone nach einem der vorhergehenden Ansprüche, **dadurch gekennzeichnet, dass** die Seite mit der Einbuchtung am Rand eine Dichtung (6) aufweist.

4. Schablone nach einem der vorhergehenden Ansprüche, **dadurch gekennzeichnet, dass** die erste Einbuchtung eine Tiefe von 2 bis 8 µm aufweist.

5. Schablone nach einem der vorhergehenden Ansprüche, **dadurch gekennzeichnet, dass** die Schablone aus Kunststoff oder Titan ist.

6. Schablone nach einem der vorhergehenden Ansprüche, **dadurch gekennzeichnet, dass** sie eine kreisförmige Massageeinrichtung aufweist.

7. Schablone nach Anspruch 6, **dadurch gekennzeichnet, dass** die Massageeinrichtung mindestens ein Ringelement aufweist.

8. Schablone nach einem der Anspruch 6 oder Anspruch 7, **dadurch gekennzeichnet, dass** die Massageeinrichtung mehrere Ringsegmente aufweist.

9. Schablone nach einem der Ansprüche 6 bis 8, **dadurch gekennzeichnet, dass** die Massageeinrichtung (38) automatisiert ist.

## Claims

1. Template for positioning on an eye, which has a circular indentation (4) on one side, which is shaped according to a desired vision correction, **characterised in that** the template (1) has a channel (5), which is connected with the indentation and **in that** a device which generates an underpressure, which changes the pressure ratios between template in a hollow space formed between the cornea and the indentation when the template is positioned on the eye is arranged at the channel in such a way that the cornea is pressed or pulled into the indentation.

2. Template according to claim 1, **characterised in that** it has a second indentation in the shape of an annular convex area around the first indentation.

3. Template according to one of the preceding claims, **characterised in that** the side with the indentation has a seal at its edge (6).

4. Template according to one of the preceding claims, **characterised in that** the first indentation has a depth of 2 to 8 µm.

5. Template according to one of the preceding claims, **characterised in that** the template is made of plastic or titanium.

6. Template according to one of the preceding claims, **characterised in that** it has a circular massage means.

7. Template according to claim 6, **characterised in that** the massage means has at least one annular element.

8. Template according to one of the claims 6 or claim 7, **characterised in that** the massage means has several annular elements.

9. Template according to one of the claims 6 to 8, **characterised in that** the massage means (38) is automated.

## Revendications

1. Gabarit à poser sur un oeil qui présente d'un côté un perçage circulaire (4), qui est formé selon une correction visuelle souhaitée, **caractérisé en ce que** le gabarit (1) présente un canal (5) qui est relié au perçage et auquel canal un dispositif est disposé lequel génère une sous-pression qui modifie le rapport de pression entre l'oeil et le gabarit dans une cavité formée entre la cornée et le perçage, lorsque le gabarit est posé sur l'oeil, de telle sorte que la cornée est enfoncée ou tirée dans le perçage.

2. Gabarit selon la revendication 1, **caractérisé en ce que** le premier perçage présente un second perçage sous la forme d'une zone incurvée circulaire.

3. Gabarit selon l'une quelconque des revendications précédentes, **caractérisé en ce que** le côté avec le perçage présente un joint (6) sur le bord.

4. Gabarit selon l'une quelconque des revendications précédentes, **caractérisé en ce que** le premier perçage présente une profondeur de 2 à 8 µm.

5. Gabarit selon l'une quelconque des revendications précédentes, **caractérisé en ce que** le gabarit est en matière plastique ou en titane.

6. Gabarit selon l'une quelconque des revendications précédentes, **caractérisé en ce qu'**il présente un dispositif de massage circulaire.

7. Gabarit selon la revendication 6, **caractérisé en ce que** le dispositif de massage présente au moins un élément annulaire.

8. Gabarit selon l'une des revendications 6 ou 7, **caractérisé en ce que** le dispositif de massage présente plusieurs éléments annulaires.

9. Gabarit selon l'une quelconque des revendications 6 à 8, **caractérisé en ce que** le dispositif de massage (38) est automatisé.
